# FASCICULE DE BREVET EUROPEEN

(11) **EP 1 521 736 B1**
(45) Date de publication et mention de la délivrance du brevet: **13.09.2017**
(21) Numéro de dépôt: 03748206.4
(22) Date de dépôt: 11.07.2003
(51) Int. Cl.: C07C 253/10, C07F 9/145, C07F 9/58, C07F 9/655, C07F 9/141, C07F 9/24, C07F 9/6571, C07F 9/6574

(54) **FABRICATION DE NITRILES A PARTIR DE COMPOSES A INSATURATION ETHYLENIQUE**
HERSTELLUNG VON NITRILEN AUS ETHYLENISCH UNGESÄTTIGTEN VERBINDUNGEN
MAKING NITRILE COMPOUNDS FROM ETHYLENICALLY UNSATURATED COMPOUNDS

(30) Priorité: 15.07.2002 FR 0208899
(43) Date de publication de la demande: 13.04.2005
(73) Titulaire: Invista Technologies S.à.r.l., 9000 St. Gallen (CH)
(72) Inventeur: GALLAND, Jean-Christophe, PRINCETON, NJ, 08540 (US); DIDILLON, Blaise, F-78740 Vaux sur Seine (FR); MARION, Philippe, F-69390 Vernaison (FR); BOURGEOIS, Damien, F-69003 Lyon (FR)
(74) Mandataire: Carpmaels & Ransford LLP
(86) Numéro de dépôt international: PCT/FR2003/002192
(87) Numéro de publication internationale: WO 2004/007432

(56) Documents cités:
- WO-A-02/13964
- DE-A- 10 052 462
- DE-A- 19 602 301
- US-B1- 6 300 515

## Description

La présente invention concerne un procédé d'hydrocyanation de composés organiques à insaturation éthylénique en composés comprenant au moins une fonction nitrile.

Elle se rapporte plus particulièrement à l'hydrocyanation de dioléfines telles que le butadiène ou d'oléfines substituées telles que des alcènes nitriles comme les pentènenitriles. L'hydrocyanation du butadiène en pentènenitriles est une réaction importante qui est mise en oeuvre industriellement depuis de nombreuses années, notamment dans le procédé de synthèse de l'adiponitrile, un grand intermédiaire chimique permettant notamment d'accéder aux monomères de nombreux polymères, dont principalement les polyamides.

Le brevet français n° 1 599 761 décrit un procédé de préparation de nitriles par addition d'acide cyanhydrique sur des composés organiques ayant au moins une double liaison éthylénique, en présence d'un catalyseur au nickel et d'une phosphite de triaryle. Cette réaction peut être conduite en présence ou non d'un solvant.

Lorsqu'un solvant est utilisé dans ce procédé de l'art antérieur, il s'agit de préférence d'un hydrocarbure, tel que le benzène ou les xylènes ou d'un nitrile tel que l'acétonitrile.

Le catalyseur mis en oeuvre est un complexe organométallique de nickel, contenant des ligands tels que les phosphines, les arsines, les stibines, les antimonites, les arsénites, les phosphites, les phosphinites ou phosphonites. Les brevets DE10052462 et WO2002/13964 décrivent également des composés organophosphites utilisés comme ligands dans les systèmes catalytiques pour la réaction d'hydrocyanation et la synthèse d'adiponitrile.

Les procédés d'hydrocyanation de diènes comprennent généralement deux étapes : une première hydrocyanation conduisant à des mononitriles insaturés ramifiés et linéaires et une seconde étape permettant d'obtenir les dinitriles. Souvent seuls les nitriles linéaires présentent un intérêt pour la synthèse de nouveaux produits comme par exemple l'adiponitrile. Ces procédés comprennent donc également une étape intermédiaire appelée étape d'isomérisation, consistant à traiter les mononitriles insaturés ramifiés pour les transformer en mononitriles insaturés linéaires.

La présence d'un promoteur pour activer le catalyseur, tel qu'un composé du bore ou un sel métallique, généralement un acide de Lewis, est également préconisée pour réaliser la seconde étape.

Le brevet FR-A-2 338 253 a proposé de réaliser l'hydrocyanation des composés ayant au moins une insaturation éthylénique, en présence d'une solution aqueuse d'un composé d'un métal de transition, notamment le nickel, le palladium ou le fer, et d'une phosphine sulfonée.

Les phosphines sulfonées décrites dans ce brevet sont des triarylphosphines sulfonées et plus particulièrement des triphénylphosphines sulfonées. Les brevets US6300515 et DE19602301 décrivent des composés biphosphites utilisés comme ligand dans des systèmes catalytiques pour des réactions d'hydroformylation ou d'isomérisation de composés allyliques. Ce procédé permet une hydrocyanation correcte, notamment du butadiène et des pentènenitriles, une séparation aisée de la solution catalytique par simple décantation et par conséquent évite au maximum le rejet d'effluents ou de déchets contenant les métaux utilisés comme catalyseur.

Toutefois, des recherches sont conduites pour trouver de nouveaux systèmes catalytiques plus performants tant en activité catalytique qu'en sélectivité et stabilité.

Un des buts de la présente invention est de proposer une nouvelle famille de ligands qui permet d'obtenir avec les métaux de transition des systèmes catalytiques présentant notamment une sélectivité améliorée en nitriles linéaires par rapport aux systèmes connus.

A cet effet, la présente invention propose un procédé d'hydrocyanation d'un composé hydrocarboné comprenant au moins une insaturation éthylénique par réaction en milieu liquide avec le cyanure d'hydrogène en présence d'un catalyseur comprenant un élément métallique qui est le nickel et un ligand organique caractérisé en ce que le ligand organique correspond à la formule générale I suivante : 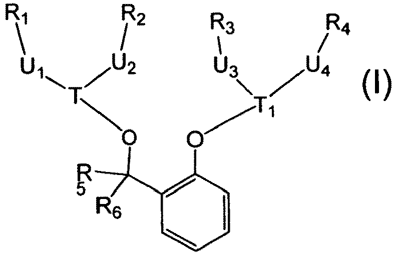 Dans laquelle :
T, T₁ identiques ou différents représentent un atome de phosphore, d'arsenic ou d'antimoine
U₁, U₂, U₃, U₄, U₅, U₆ identiques ou différents représentent un atome d'oxygène ou un radical NR, R représentant un radical alkyle, aryle, sulfonyle ou carbonylé,
R₁, R₂, R₃, R₄ identiques ou différents représentent un radical aromatique, aliphatique ou cycloaliphatique substitué ou non comprenant un ou plusieurs cycles sous forme condensée ou non et pouvant comprendre un ou plusieurs hétéroatomes, les radicaux R₁ et R₂ d'une part, et R₃ et R₄ d'autre part peuvent être reliés entre eux par une liaison covalente, une chaîne hydrocarbonée ou un hétéroatome, et quand l'un des radicaux U₁, U₂, U₃, U₄ comprend un atome N, le radical R₁, R₂, R₃, R₄ associé peut former un cycle incluant l'élément N dudit radical,
R₅, R₆ identiques ou différents représentent des radicaux hydrocarbonés aliphatiques, cycloaliphatiques ou aromatiques comprenant de 1 à 12 atomes de carbone, R₅, R₆ représentant également l'atome d'hydrogène,
les composés organiques comportant au moins une double liaison éthylénique étant choisis parmi le butadiène, l'isoprène, l'hexadiène-1,5, le cyclooctadiène-1,5, le pentène-3-nitrile, le pentène-4-nitrile, le styrène, le méthylstyrène, le vinylnaphtalène, le cyclohexène, le méthylcyclohexène ainsi que les mélanges de plusieurs de ces composés.

Comme exemples de ligands convenables pour l'invention, on peut citer les composés suivants listés dans le tableau I ci-dessous :

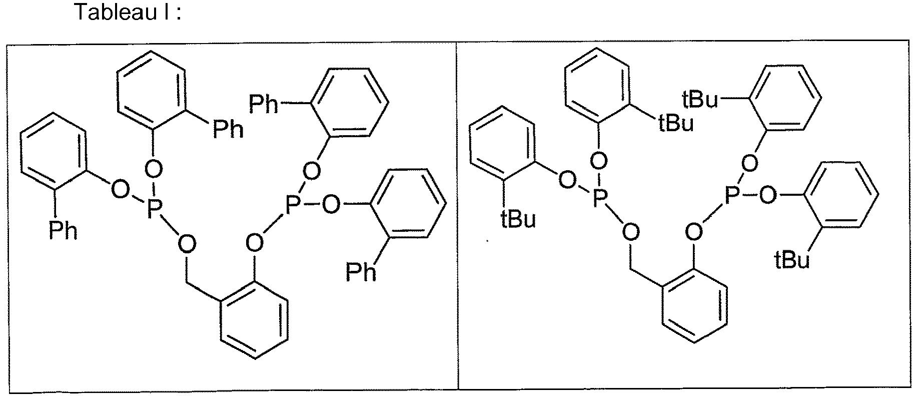

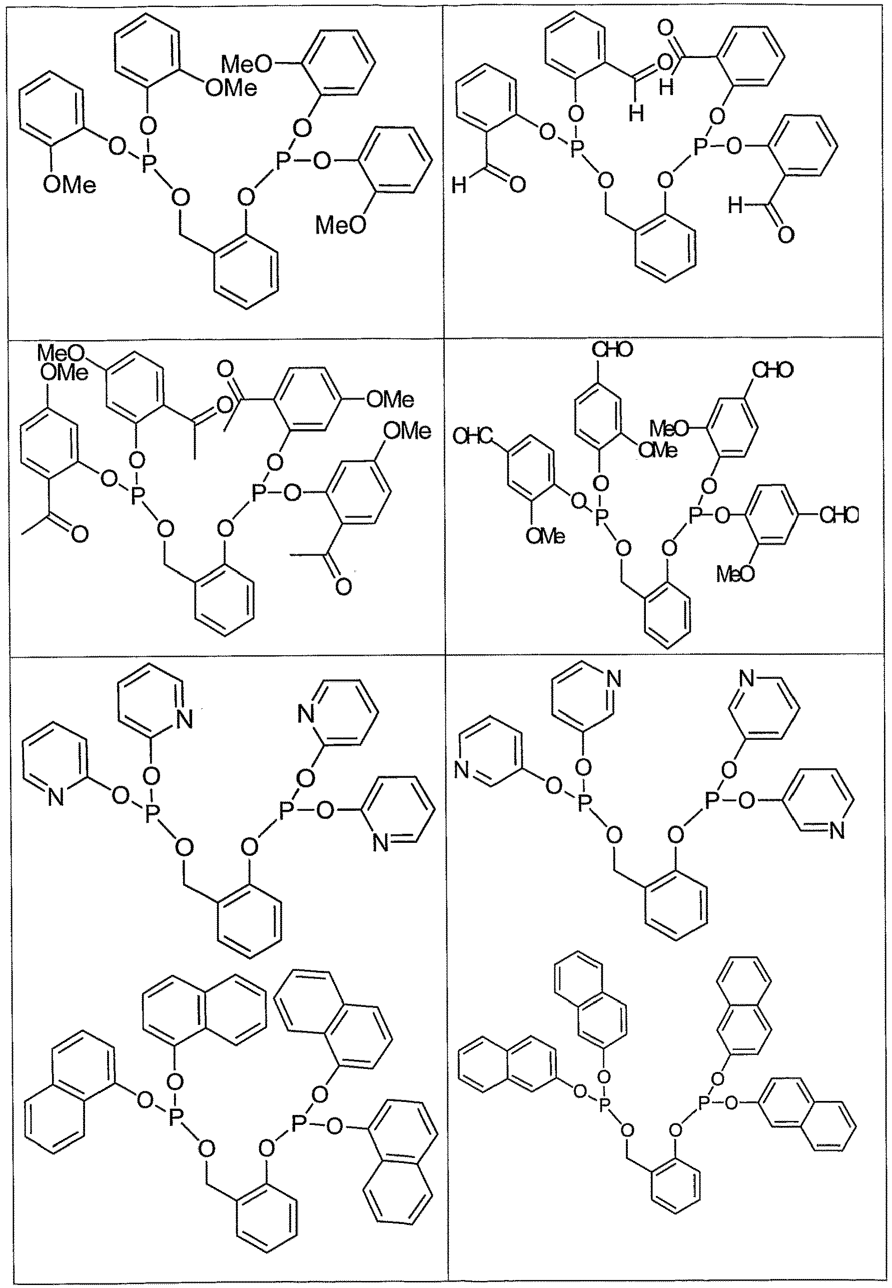

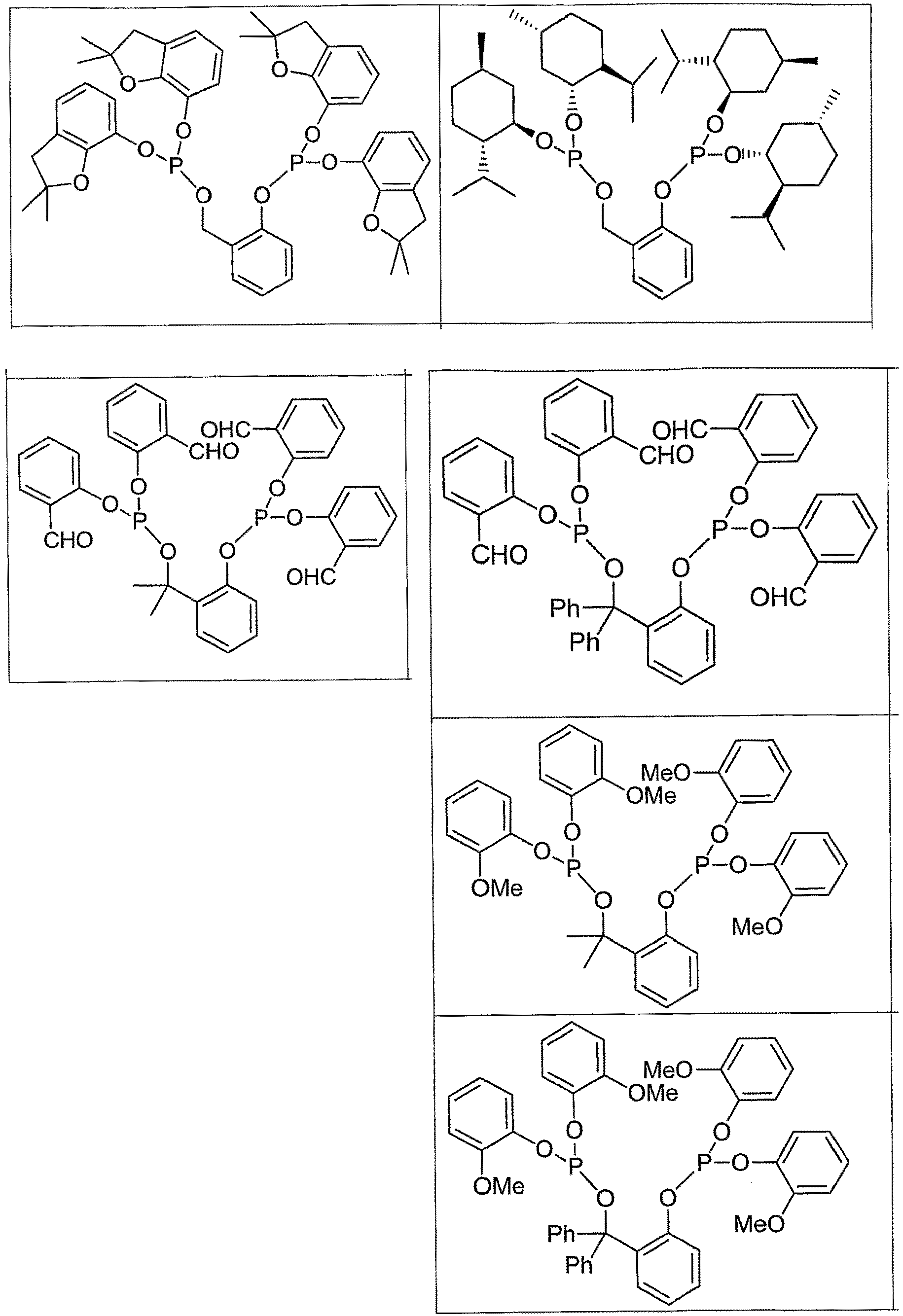

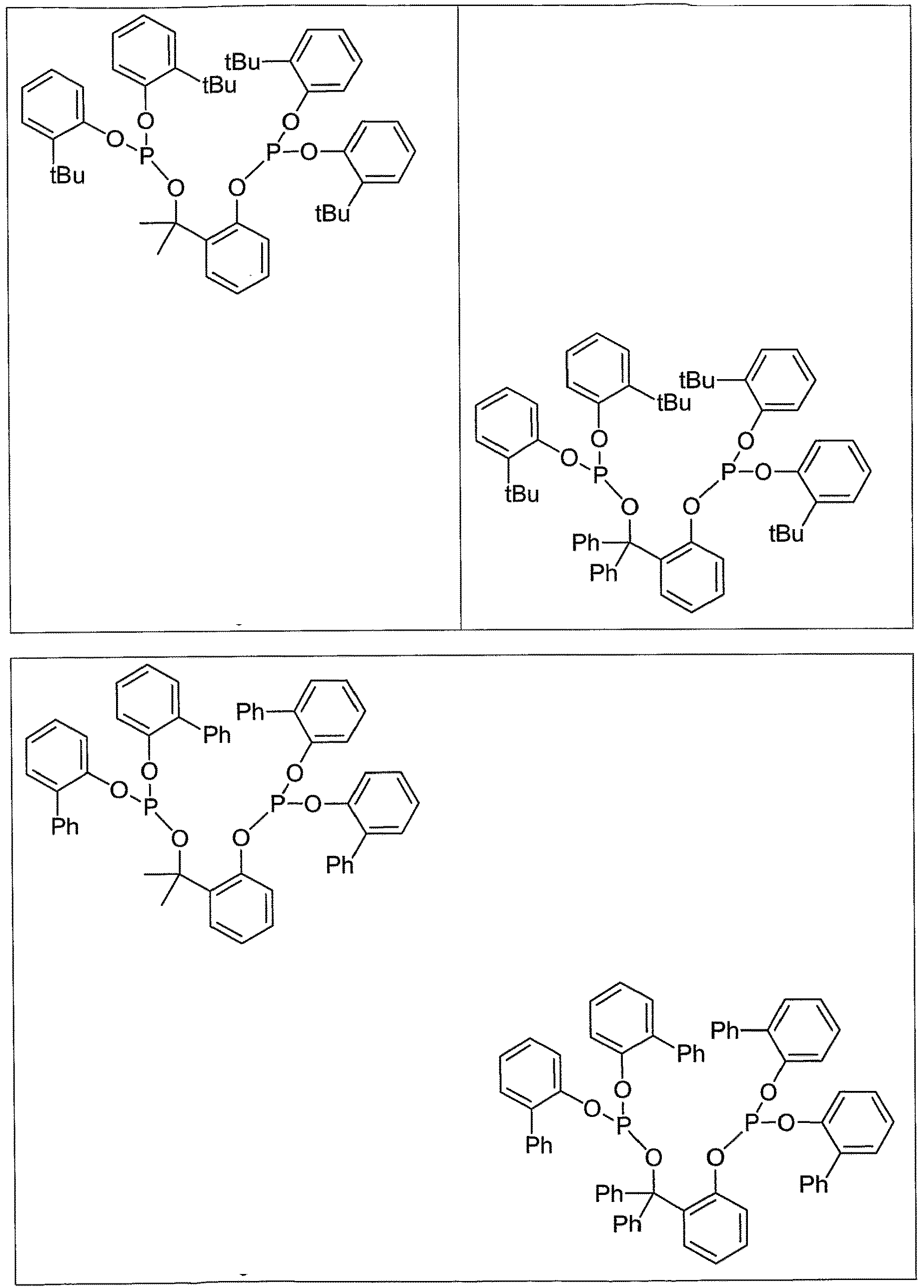

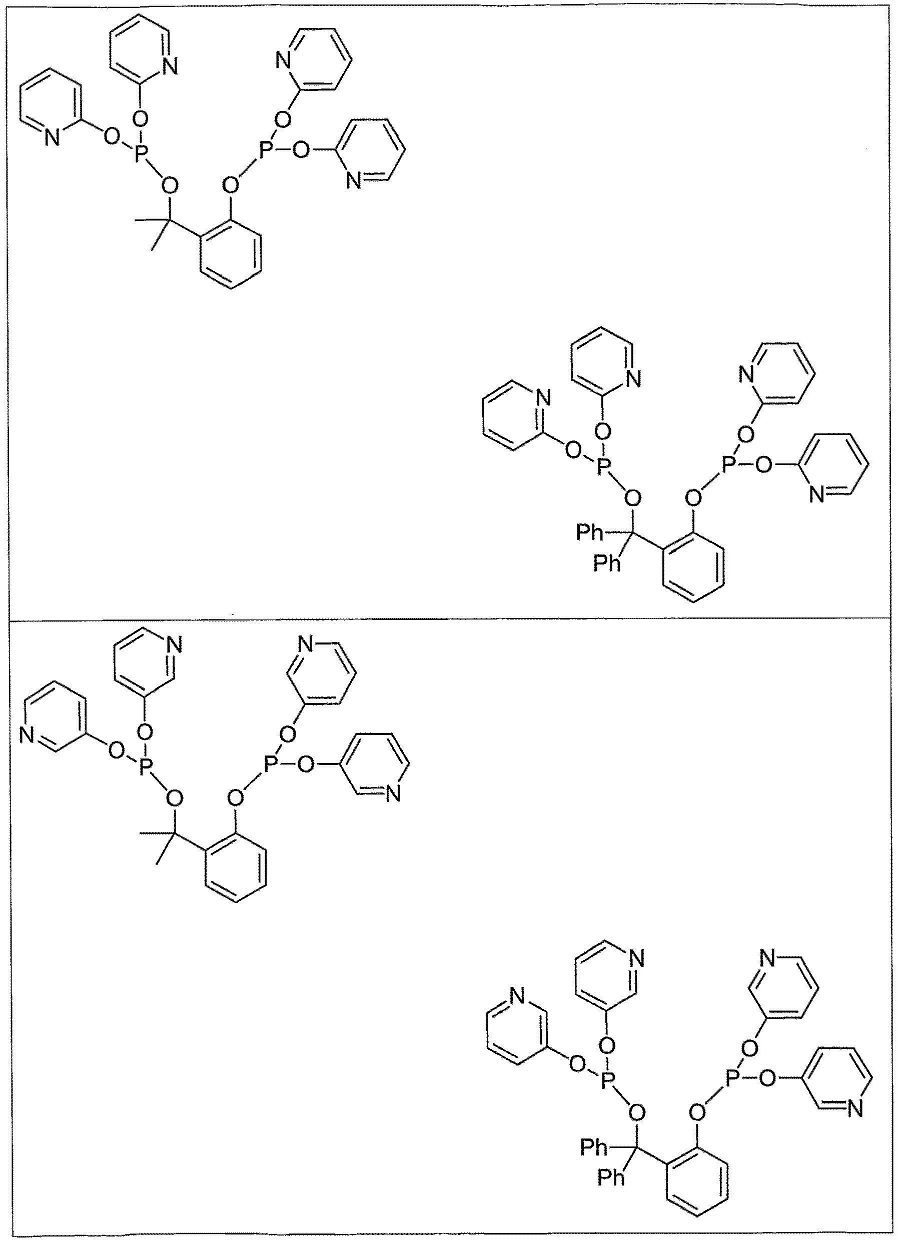

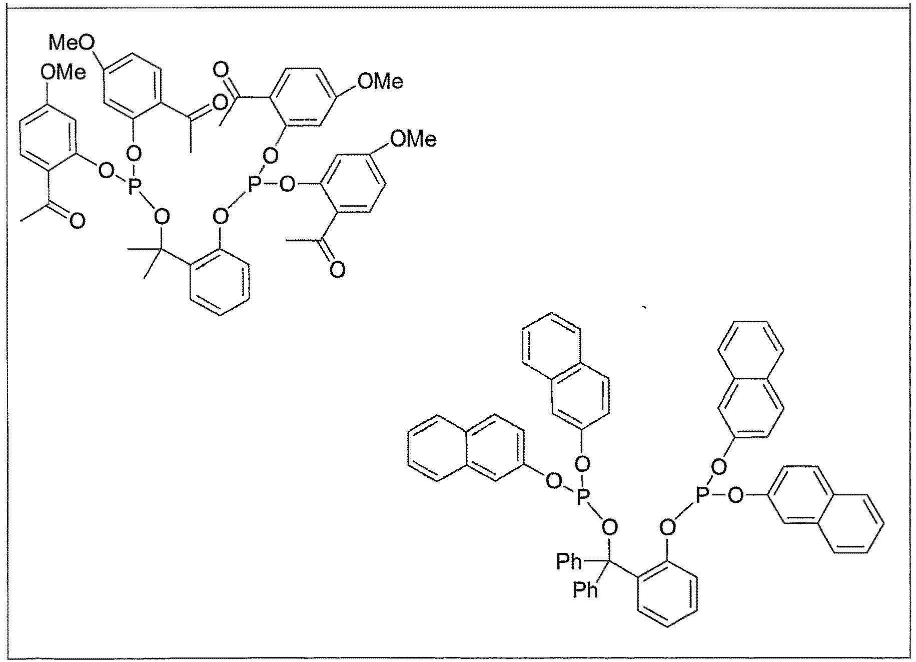

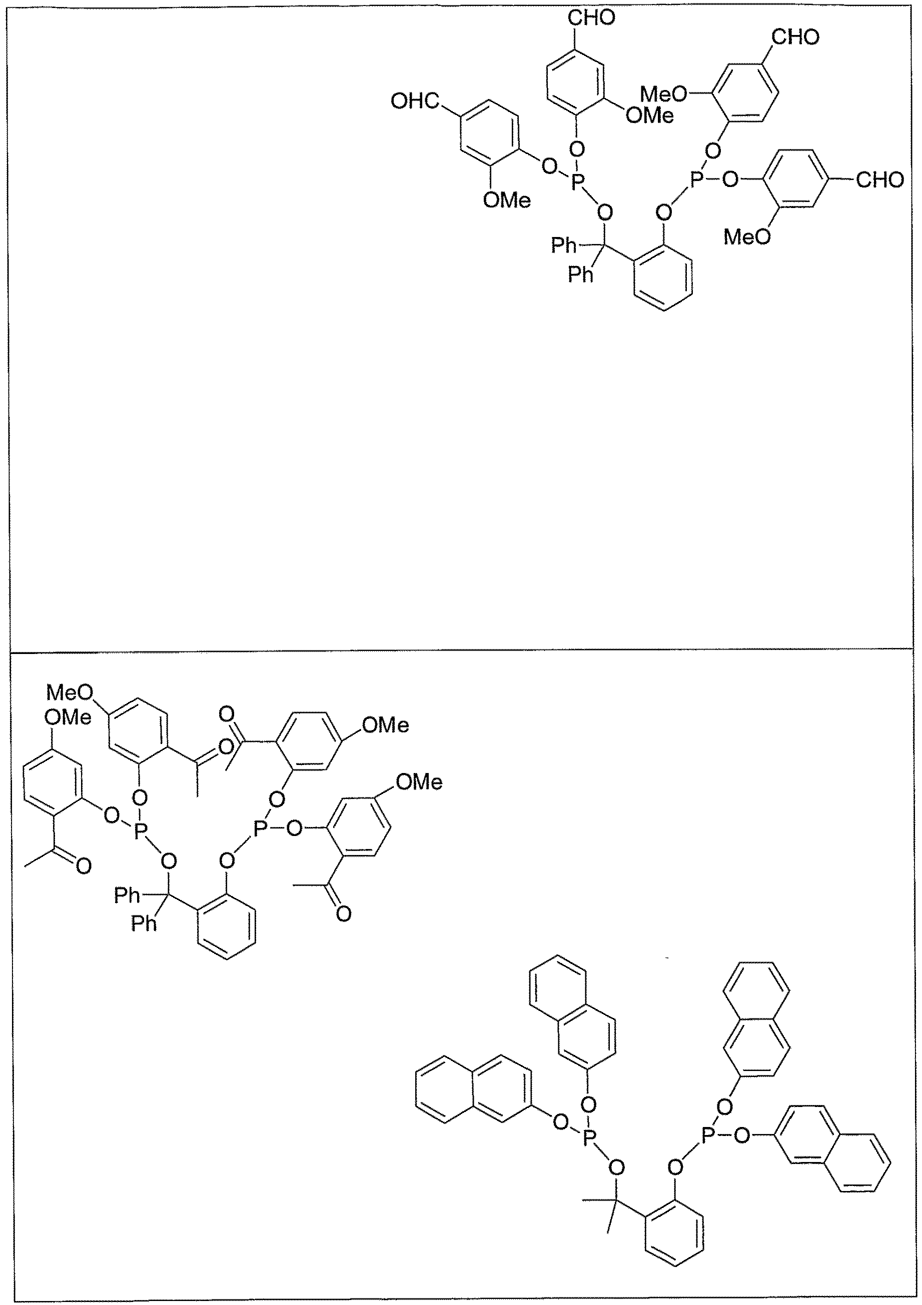

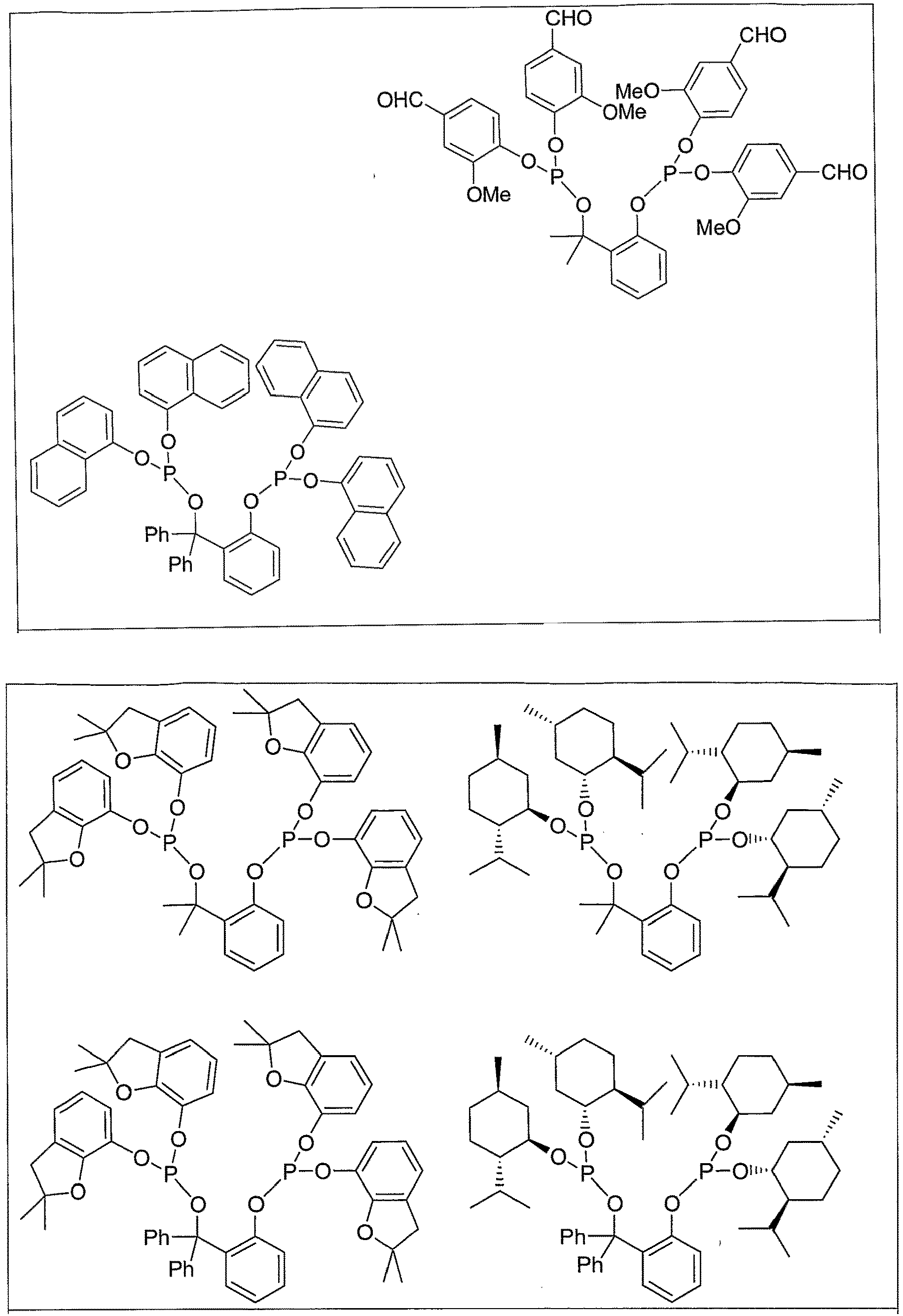

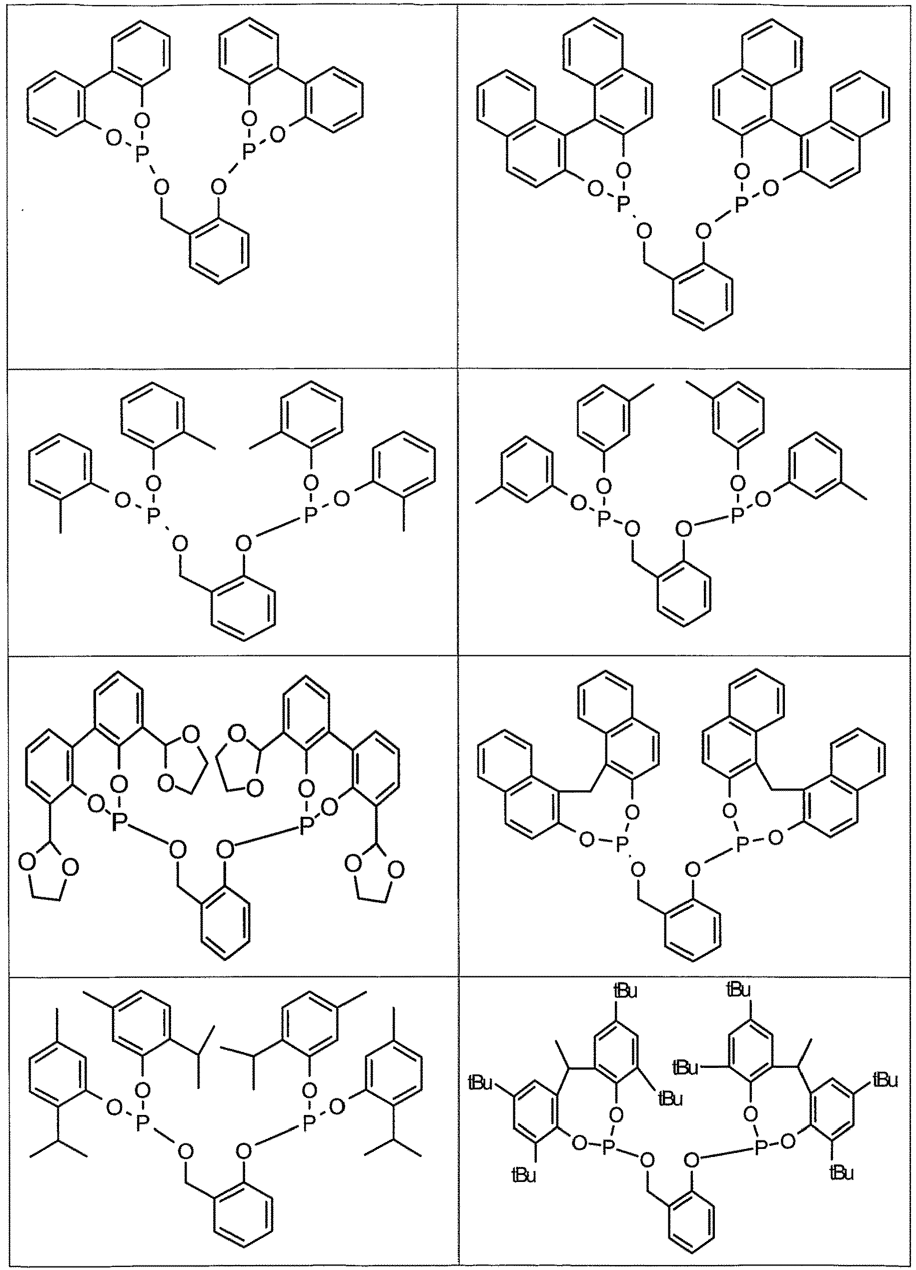

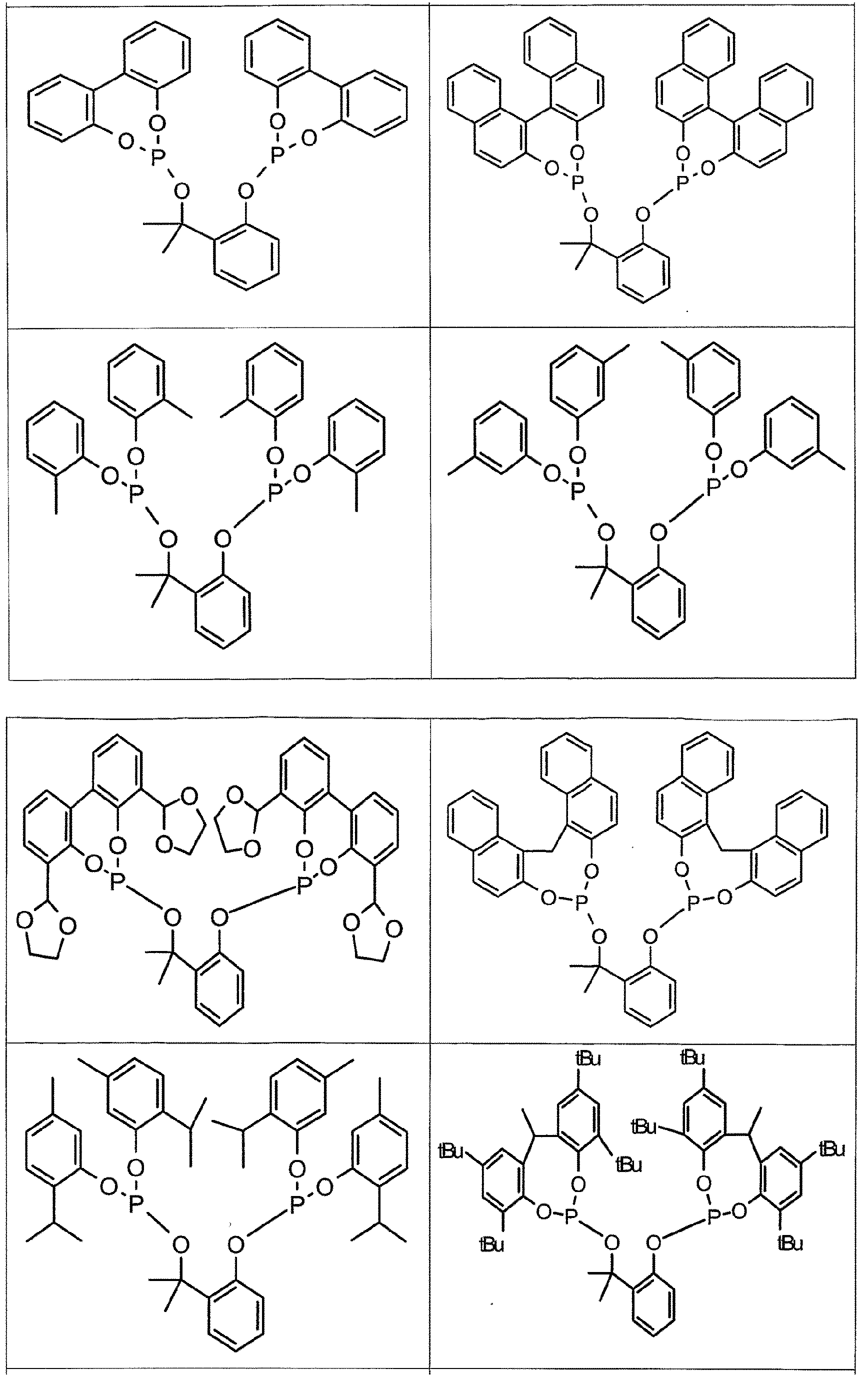

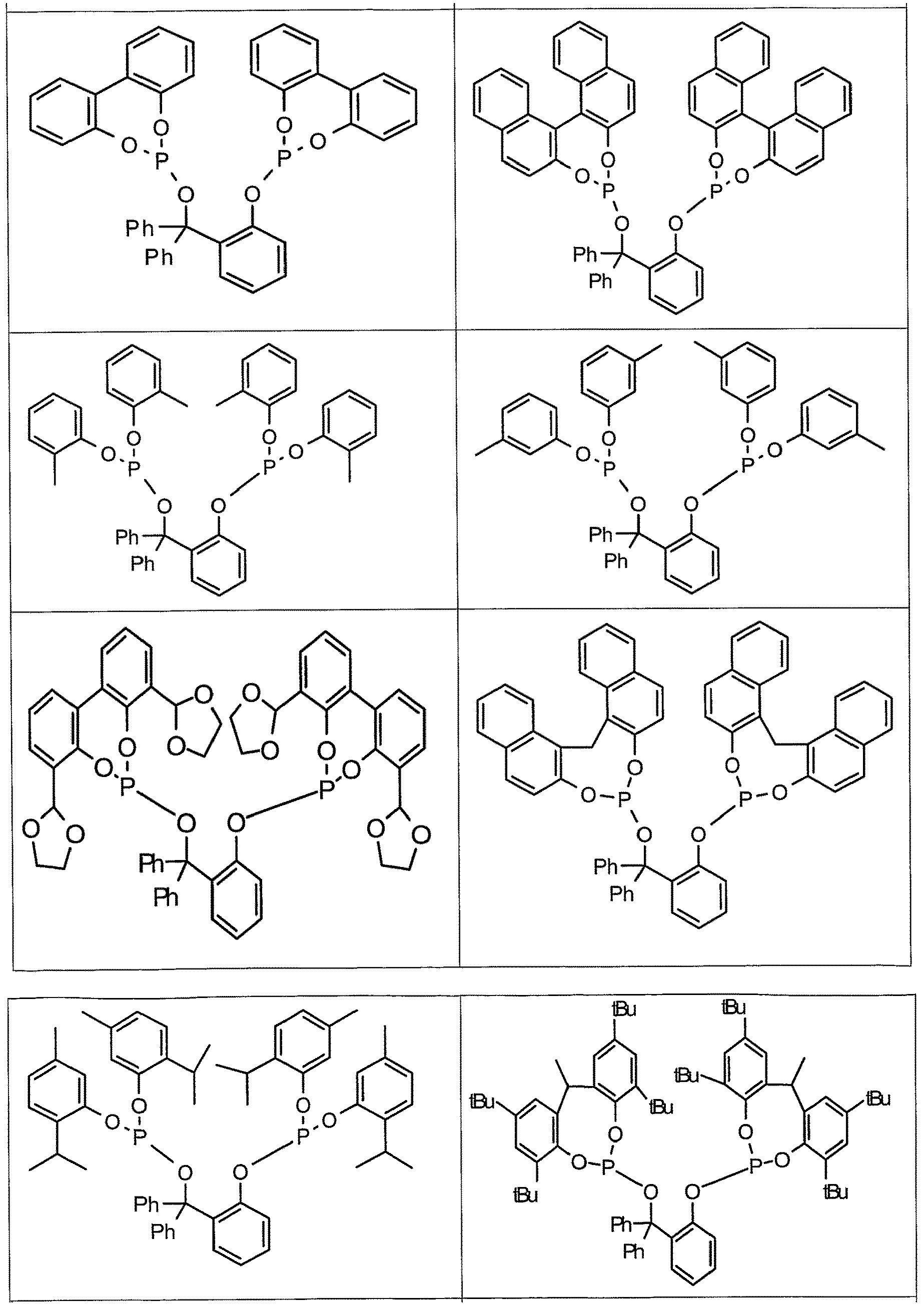

Dans lesquelles :
tBu représente le groupe tertiobutyle
Me représente le groupe méthyle
Ph représente le groupe phényle

Selon l'invention, le catalyseur correspond, avantageusement, à la formule générale (V) :

M [L_{f}]ᵥ (V)

Dans laquelle :
M est le nickel
L_{f} représente le ligand organique de formule (I)
v représente un nombre compris entre 1 et 4 (bornes incluses)

La préparation des complexes organométalliques comprenant les ligands organiques de l'invention peut être effectuée en mettant en contact une solution d'un composé du nickel avec une solution du ligand organique de l'invention.

Le composé du nickel peut être dissous dans un solvant.

Le nickel peut se trouver dans le composé mis en oeuvre, soit au degré d'oxydation qu'il aura dans le complexe organométallique, soit à un degré d'oxydation supérieur.

A titre d'exemple, on peut indiquer que dans les complexes organométalliques de l'invention, le nickel est au degré d'oxydation (0).

Si lors de la préparation du complexe organométallique, le nickel est mis en oeuvre à un degré d'oxydation plus élevé, il pourra être réduit in situ.

Les complexes organométalliques comprenant les ligands organiques de l'invention peuvent être utilisés comme catalyseurs dans les réactions d'hydrocyanation d'oléfines.

On peut citer à titre d'exemples non limitatifs :
- les composés dans lesquels le nickel est au degré d'oxydation zéro comme le tétracyanonickelate de potassium K₄[Ni(CN)₄], le bis(acrylonitrile)nickel zéro, le bis(cyclooctadiène-1,5)nickel zéro (appelé également Ni(cod)₂) et les dérivés contenant des ligands comme le tétrakis(triphénylphosphine)nickel zéro.
- les composés du nickel comme les carboxylates (notamment l'acétate), carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl-et alkyl-sulfonates.

Quand le composé du nickel utilisé correspond à un état d'oxydation du nickel supérieur à 0, on ajoute au milieu réactionnel un réducteur du nickel réagissant préférentiellement avec celui-ci dans les conditions de la réaction. Ce réducteur peut être organique ou minéral. On peut citer comme exemples non limitatifs les borohydrures comme le BH₄Na, le BH₄K, la poudre de Zn, le magnésium ou l'hydrogène.

Quand le composé du nickel utilisé correspond à l'état d'oxydation 0 du nickel, on peut également ajouter un réducteur du type de ceux précités, mais cet ajout n'est pas impératif.

Les composés organiques comportant au moins une double liaison éthylénique sont le butadiène, l'isoprène, l'hexadiène-1,5, le cyclooctadiène-1,5, le pentène-3-nitrile, le pentène-4-nitrile, le styrène, le méthylstyrène, le vinylnaphtalène, le cyclohexène, le méthylcyclohexène ainsi que les mélanges de plusieurs de ces composés.

Les pentènenitriles notamment peuvent contenir des quantités, généralement minoritaires, d'autres composés, comme le méthyl-2-butène-3-nitrile, le méthyl-2-butène-2-nitrile, le pentène-2-nitrile, le valéronitrile, l'adiponitrile, le méthyl-2-glutaronitrile, l'éthyl-2-succinonitrile ou le butadiène, provenant par exemple de la réaction antérieure d'hydrocyanation du butadiène en nitriles insaturés.

En effet, lors de l'hydrocyanation du butadiène, il se forme avec les pentènenitriles linéaires des quantités non négligeables de méthyl-2-butène-3-nitrile et de méthyl-2-butène-2-nitrile.

Le système catalytique utilisé pour l'hydrocyanation selon le procédé de l'invention peut être préparé avant son introduction dans la zone de réaction, par exemple par addition à du ligand conforme à l'invention seul ou dissout dans un solvant, la quantité appropriée de composé du nickel et éventuellement du réducteur. Il est également possible de préparer le système catalytique "in situ" par simple addition du ligand et du composé du nickel dans le milieu réactionnel d'hydrocyanation avant ou après l'addition du composé à hydrocyaner.

La quantité de composé du nickel utilisée est choisie pour obtenir une concentration en mole de nickel par mole de composés organiques à hydrocyaner ou isomériser comprise entre 10⁻⁴ et 1, et de préférence entre 0,005 et 0,5 mole de nickel mis en oeuvre.

La quantité de ligands organiques de l'invention utilisée pour former le catalyseur est choisie de telle sorte que le nombre de moles de ce composé rapporté à 1 mole de nickel soit de 0,5 à 50 et de préférence de 2 à 10.

Bien que la réaction soit conduite généralement sans solvant, il peut être avantageux de rajouter un solvant organique inerte. Le solvant peut être un solvant du catalyseur qui est miscible à la phase comprenant le composé à hydrocyaner à la température d'hydrocyanation. A titre d'exemples de tels solvants, on peut citer les hydrocarbures aromatiques, aliphatiques ou cycloaliphatiques.

Ce solvant peut également être partiellement miscible avec les composés à hydrocyaner, notamment quand le milieu réactionnel est à une température inférieure à la température de réaction. Ainsi, on peut, à de telles températures, obtenir un système biphasique. Dans le cas où le système catalytique est soluble dans ledit solvant, son extraction du milieu réactionnel en est facilitée. De tels solvants partiellement miscibles ou non miscibles peuvent être l'eau ou des sels organiques fondus à caractère ionique. De tels solvants sont utilisés notamment quand le ligand organique comprend des radicaux anioniques le rendant soluble dans les milieux ioniques. Ces radicaux sont par exemple des groupements sulfonates, carbonates, carboxylates, phosphates, ammonium, guanidinium, imidazolium, substituant les radicaux aromatiques du ligand.

La réaction d'hydrocyanation est généralement réalisée à une température de 10 °C à 200 °C et de préférence de 30 °C à 120 °C. Elle est avantageusement réalisée en milieu monophasique, à la température de réaction.

Le procédé de l'invention peut être mis en oeuvre de manière continue ou discontinue.

Le cyanure d'hydrogène mis en oeuvre peut être préparé à partir des cyanures métalliques, notamment le cyanure de sodium, ou des cyanhydrines, comme la cyanhydrine de l'acétone ou par tout autre procédé de synthèse connu.

Le cyanure d'hydrogène est introduit dans le réacteur sous forme gazeuse, de mélange de gaz ou sous forme liquide. Il peut également être préalablement dissous dans un solvant organique.

Dans le cadre d'une mise en oeuvre discontinue, on peut en pratique charger dans un réacteur, préalablement purgé à l'aide d'un gaz inerte (tel qu'azote, argon), soit une solution contenant la totalité ou une partie des divers constituants tels que le ligand organique de l'invention, le composé du nickel, les éventuels réducteurs et solvants, soit séparément lesdits constituants. Généralement le réacteur est alors porté à la température choisie, puis le composé à hydrocyaner est introduit. Le cyanure d'hydrogène est alors lui-même introduit, de préférence de manière continue et régulière.

Quand la réaction (dont on peut suivre l'évolution par dosage de prélèvements) est terminée, le mélange réactionnel est soutiré après refroidissement et les produits de la réaction sont isolés, par exemple, par distillation.

Un perfectionnement au procédé d'hydrocyanation de composés à insaturation éthylénique selon la présente invention concerne notamment l'hydrocyanation des composés nitriles à insaturation éthylénique choisis parmi le pentène-3-nitrile, le pentène-4-nitrile et leurs mélanges, par réaction avec le cyanure d'hydrogène et consiste à utiliser un système catalytique conforme à la présente invention avec un cocatalyseur consistant en au moins un acide de Lewis.

Les pentènenitriles provenant de la réaction anterieure d'hydrocyanation du butadiène et/ou de l'isomérisation du méthyl-2-butène-3-nitrile en pentènenitriles peuvent contenir des quantités, généralement minoritaires, d'autres composés, comme le méthyl-2-butène-3-nitrile, le méthyl-2-butène-2-nitrile, le pentène-2-nitrile, le valéronitrile, l'adiponitrile, le méthyl-2-glutaronitrile, l'éthyl-2-succinonitrile ou le butadiène, provenant de la réaction antérieure d'hydrocyanation du butadiène et/ou de l'isomérisation du méthyl-2-butène-3-nitrile en pentènenitriles.

L'acide de Lewis utilisé comme cocatalyseur permet notamment, dans le cas de l'hydrocyanation des nitriles aliphatiques à insaturation éthylénique, d'améliorer la linéarité des dinitriles obtenus, c'est-à-dire le pourcentage de dinitriles linéaires par rapport à la totalité des dinitriles formés, et/ou d'augmenter l'activité et la durée de vie du catalyseur.

Par acide de Lewis, on entend dans le présent texte, selon la définition usuelle, des composés accepteurs de doublets électroniques.

On peut mettre en oeuvre notamment les acides de Lewis cités dans l'ouvrage édité par G. A. OLAH "Friedel-Crafts and related Reactions", tome 1, pages 191 à 197 (1963).

Les acides de Lewis qui peuvent être mis en oeuvre comme cocatalyseurs dans le présent procédé sont choisis parmi les composés des éléments des groupes Ib, IIb, IIIa, IIIb, IVa, IVb, Va, Vb, VIb, VIIb et VIII de la classification périodique des éléments. Ces composés sont le plus souvent des sels, notamment des halogénures, comme chlorures ou bromures, sulfates, sulfonates, halogénoalkylsulfonates, perhalogénoalkylsulfonates, notamment fluoroalkylsulfonates ou perfluoroalkylsulfonates, les halogénoalkylacétates, les perhalogénoalkylacétates, notamment fluoroalkylacétates ou perfluoroalkylacétates, les carboxylates et phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, le trifluoroacétate d'indium, le trifluoroacétate de zinc, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium.

On peut également utiliser comme acide de Lewis des composés organométalliques comme le triphénylborane, l'isopropylate de titane.

On peut bien entendu mettre en oeuvre des mélanges de plusieurs acides de Lewis.

Parmi les acides de Lewis, on préfère tout particulièrement le chlorure de zinc, le bromure de zinc, le chlorure stanneux, le bromure stanneux le triphénylborane et les mélanges chlorure de zinc/chlorure stanneux, le trifluorométhylsulfonate d'indium, le trifluoroacétate d'indium, le trifluoroacétate de zinc.

Le cocatalyseur acide de Lewis mis en oeuvre représente généralement de 0,01 à 50 moles par mole de composé du nickel, et de préférence de 0,5 à 10 mole par mole.

Comme pour la mise en oeuvre du procédé de base de l'invention, la solution catalytique utilisée pour l'hydrocyanation en présence d'acide de Lewis peut être préparée avant son introduction dans la zone de réaction, par exemple par addition au milieu réactionnel du ligand de formule (I), de la quantité appropriée de composé du nickel, de l'acide de Lewis et éventuellement du réducteur. Il est également possible de préparer la solution catalytique"in situ"par simple mélange de ces divers constituants.

Il est également possible dans les conditions du procédé d'hydrocyanation de la présente invention, et notamment en opérant en présence du catalyseur décrit précédemment comportant au moins un ligand de formule (I) et au moins un composé du nickel, de réaliser, en absence de cyanure d'hydrogène, l'isomérisation du méthyl-2-butène-3-nitrile en pentènenitriles, et plus généralement des nitriles insaturés ramifiés en nitriles insaturés linéaires.

Le méthyl-2-butène-3-nitrile soumis à l'isomérisation selon l'invention peut être mis en oeuvre seul ou en mélange avec d'autres composés.

Ainsi on peut engager du méthyl-2-butène-3-nitrile en mélange avec du méthyl-2-butène-2 nitrile, du pentène-4-nitrile, du pentène-3-nitrile, du pentène-2-nitrile, du butadiène, de l'adiponitrile, du méthyl-2-glutaronitrile, de l'éthyl-2-succinonitrile ou du valéronitrile.

Il est particulièrement intéressant de traiter le mélange réactionnel provenant de l'hydrocyanation du butadiène par HCN en présence d'au moins un ligand de formule (I) et d'au moins un composé du nickel, plus préférentiellement d'un composé du nickel au degré d'oxydation 0, tel que défini précédemment.

Dans le cadre de cette variante préférée, le système catalytique étant déjà présent pour la réaction d'hydrocyanation du butadiène, il suffit d'arrêter toute introduction de cyanure d'hydrogène, pour laisser se produire la réaction d'isomérisation.

On peut, le cas échéant, dans cette variante faire un léger balayage du réacteur à l'aide d'un gaz inerte comme l'azote ou l'argon par exemple, afin de chasser l'acide cyanhydrique qui pourrait être encore présent.

La réaction d'isomérisation est généralement réalisée à une température de 10 °C à 200 °C et de préférence de 60 °C à 160 °C.

Dans le cas préféré d'une isomérisation suivant immédiatement la réaction d'hydrocyanation du butadiène, il sera avantageux d'opérer à la température à laquelle l'hydrocyanation a été conduite.

Comme pour le procédé d'hydrocyanation de composés à insaturation éthylénique, le système catalytique utilisé pour l'isomérisation peut être préparé avant son introduction dans la zone de réaction, par exemple par addition dans un solvant du ligand de formule (I), de la quantité appropriée de composé du nickel et éventuellement du réducteur. Il est également possible de préparer le système catalytique "in situ" par simple addition de ces divers constituants dans le milieu réactionnel. La quantité du nickel utilisée, ainsi que la quantité de ligand de formule (I) sont les mêmes que pour la réaction d'hydrocyanation.

Bien que la réaction d'isomérisation soit conduite généralement sans solvant, il peut être avantageux de rajouter un solvant organique inerte qui pourra être celui de l'extraction ultérieure. C'est notamment le cas lorsqu'un tel solvant a été mis en oeuvre dans la réaction d'hydrocyanation du butadiène ayant servi à préparer le milieu soumis à la réaction d'isomérisation. De tels solvants peuvent être choisis parmi ceux qui ont été cités précédemment pour l'hydrocyanation.

La préparation de composés dinitriles par hydrocyanation d'une oléfine comme le butadiène peut être réalisée en utilisant un système catalytique conforme à l'invention pour les étapes de formation des mononitriles insaturés et l'étape d'isomérisation ci-dessus, la réaction d'hydrocyanation des mononitriles insaturés en dinitriles pouvant être mis en oeuvre avec un système catalytique conforme à l'invention ou tout autre système catalytique déjà connu pour cette réaction.

De même, la réaction d'hydrocyanation de l'oléfine en mononitriles insaturés et l'isomérisation de ceux-ci peuvent être réalisées avec un système catalytique différent de celui de l'invention, l'étape d'hydrocyanation des mononitriles insaturés en dinitriles étant mis en oeuvre avec un système catalytique conforme à l'invention.

Les exemples qui suivent illustrent l'invention.

Dans les exemples les abréviations utilisées ont les significations indiquées ci-dessous.
cod : 1, 5-cyclooctadiène.
eq : équivalent.
3PN : 3-pentènenitrile.
4PN : 4-pentènenitrile.
3+4PN : 3PN + 4PN.
TT (Y) : taux de transformation du produit à hydrocyaner Y correspondant au rapport du nombre de moles transformées de Y sur le nombre de moles initiales de Y.
Linéarité (L) : rapport du nombre de moles d'adiponitrile (AdN) formées au nombre de moles de dinitriles formées (somme des moles de AdN, éthylsuccinonitrile (ESN) et méthylglutaronitrile (MGN)).
CPG : chromatographie phase gazeuse.
ml : millilitre.
mol : mole.
mmol : millimole.

Les différents produits conformes à l'invention peuvent être préparés selon l'un des deux modes opératoires suivants :

### Mode opératoire I :

On utilise un phosphochloridite préparé à partir d'un dérivé du phénol ou du biphénol et de PCl₃ selon un mode opératoire classique (voir par exemple la méthode décrite par G. Buisman et Al. dans Tetrahedron, Asymmetry, vol 4, (7), pp 1625-1634 (1993)), et un diol disponible commercialement. La procédure générale suivante est représentative :
Sous argon, dans un réacteur de 100 ml sont dissous 6 mmol de phosphorochloridite dans 20 ml de toluène anhydre. La solution est agitée à -10°C. Une solution de 3 mmol de diol et de 10 mmol de triéthylamine dans 20 ml de toluène anhydre est introduite goutte à goutte dans le milieu réactionnel maintenu à -10°C : un précipité blanc se forme. La suspension est maintenue sous agitation vigoureuse pendant 18 h à 25°C, puis filtrée sous argon sur un lit d'alumine basique. Après rinçage au toluène, le filtrat est concentré sous pression réduite pour conduire au produit désiré, utilisé sans autre purification.

Les ligands suivants ont été préparés selon le mode opératoire décrit ci-dessus :

| Exemple | Ligand | Structure | Phosphochloridite | Diol |
|---|---|---|---|---|
| 1 | **A** | 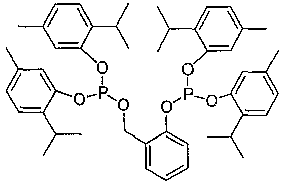 | 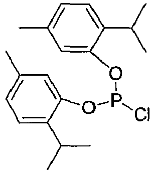 | 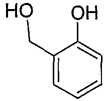 |
| 2 | **B** | 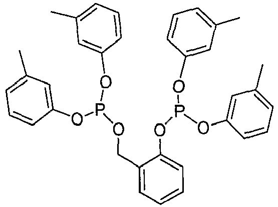 | 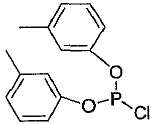 | 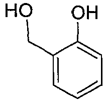 |
| 3 | **C** | 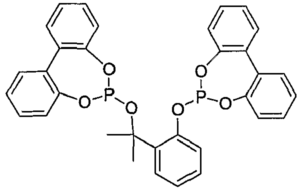 | 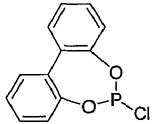 | 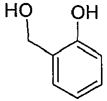 |

### Mode opératoire II :

On utilise un phosphoramidite préparé à partir de Cl₂PNEt₂ (disponible commercialement) et d'un phénol substitué ou non selon le mode opératoire suivant :
A un mélange de Cl₂PNEt₂ (0.20 mol) et de triéthylamine (0.44 mol) dans du toluène (800 ml) à 0°C, on ajoute lentement sous forte agitation une solution du phénol (0.40 mol) dans du toluène (100 ml). On observe la formation d'un précipité blanc. On laisse remonter à température ambiante et on agite toujours vigoureusement pendant 2 h. Le mélange est ensuite filtré sur un lit de silice et concentré sous vide pour donner le phosphoramidite (ArO)₂PNEt₂ avec une pureté supérieure à 95%.

On utilise par ailleurs un diol disponible commercialement. La procédure générale suivante est représentative :
Sous argon, dans un réacteur de 100 ml sont introduit 6 mmol de phosphoramidite dans 20 ml de toluène anhydre. La solution est agitée à 0°C, et on y ajoute 7.5 ml d'une solution 2M d'acide chlorhydrique dans l'éther en 30 min. On observe la formation d'un précipité blanc, et on agite 1 h à température ambiante. Une solution de 3 mmol de diol et de 10 mmol de triéthylamine dans 20 ml de toluène anhydre est ensuite introduite goutte à goutte dans le milieu réactionnel refroidi à -10°C. La suspension est maintenue sous agitation vigoureuse pendant 18 h à 25°C, puis filtrée sous argon sur un lit d'alumine basique. Après rinçage au toluène, le filtrat est concentré sous pression réduite pour conduire au produit désiré, utilisé sans autre purification.

Les ligands suivants ont été préparés selon le mode opératoire décrit ci-dessus :

| Exemple | Ligand | Structure | Phosphoramidite | Diol |
|---|---|---|---|---|
| 4 | **D** | 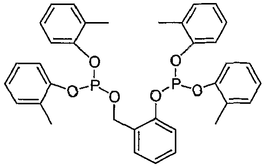 | 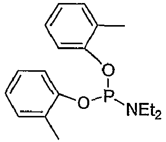 | 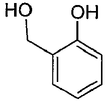 |
| 5 | **E** | 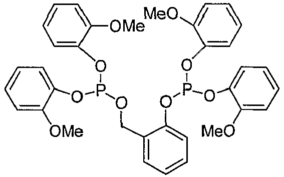 | 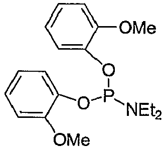 | 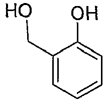 |
| 6 | **F** | 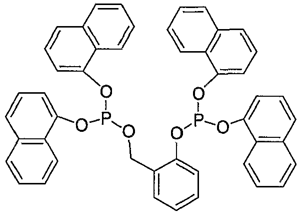 | 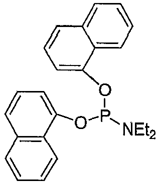 | 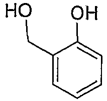 |
| 7 | **G** | 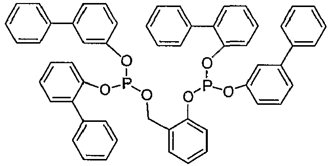 | 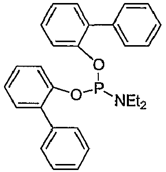 | 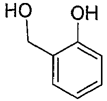 |

### Exemples d'hydrocyanation du 3-pentènenitrile (3PN) en adiponitrile (AdN).

Le mode opératoire général utilisé est le suivant :
Sous atmosphère d'argon, dans un tube en verre type Shott de 60 ml équipé d'un bouchon-septum, sont chargés successivement
   - le ligand (2,5 eq),
   - 1.21 g (15 mmol ; 30 eq) de 3PN anhydre,
   - 138 mg (0,5 mmol ; 1 eq) de Ni(cod)₂ et
   - L'acide de Lewis (0,5 mmol ; 1 eq).

Le mélange est porté, sous agitation, à 70°C. La cyanhydrine de l'acétone est injectée dans le milieu réactionnel par un pousse-seringue à un débit de 0,45 ml par heure. Après 3 heures d'injection, le pousse-seringue est stoppé. Le mélange est refroidi à température ambiante, dilué à l'acétone et analysé par chromatographie en phase gazeuse.

Les résultats sont regroupés dans le tableau suivant :

| Exemple | Ligand | Acide de Lewis | TT (3PN) | Linéarité |
|---|---|---|---|---|
| 8 | **A** | ZnCl₂ | 64% | 70% |
| 9 | **B** | ZnCl₂ | 14% | 71% |
| 10 | **C** | ZnCl₂ | 20% | 73% |
| 11 | **D** | ZnCl₂ | 44% | 76% |
| 12 | **E** | ZnCl₂ | 11% | 72% |
| 13 | **E** | CoCl₂ | 32% | 74% |
| 14 | **F** | ZnCl₂ | 16% | 67% |
| 15 | **G** | ZnCl₂ | 48% | 80% |
| 16 | **G** | YCl₃ | 64% | 88% |

## Revendications

1. Procédé d'hydrocyanation d'un composé hydrocarboné comprenant au moins une insaturation éthylénique par réaction en milieu liquide avec le cyanure d'hydrogène en présence d'un catalyseur comprenant un élément métallique qui est le nickel et un ligand organique, **caractérisé en ce que** le ligand organique correspond à la formule générale I suivante : 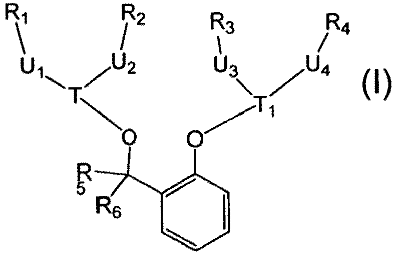 dans laquelle :
T, T₁ identiques ou différents représentent un atome de phosphore, d'arsenic ou d'antimoine
U₁, U₂, U₃, U₄ identiques ou différents représentent un atome d'oxygène ou un radical NR, R représentant un radical alkyle, aryle, sulfonyl ou carbonylé,
R₁, R₂, R₃, R₄ identiques ou différents représentent un radical aromatique, aliphatique ou cycloaliphatique substitué ou non comprenant un ou plusieurs cycles sous forme condensée ou non et pouvant comprendre un ou plusieurs hétéroatomes, les radicaux R₁ et R₂ d'une part, et R₃ et R₄ d'autre part peuvent être reliés entre eux par une liaison covalente, une chaîne hydrocarbonée ou un hétéroatome, et quand l'un des radicaux U₁, U₂, U₃, U₄ comprend un atome N, le radical R₁, R₂, R₃, R₄ associé peut former un cycle incluant l'élément N dudit radical, et
R₅, R₆ identiques ou différents représentent des radicaux hydrocarbonés aliphatiques, cycloaliphatiques ou aromatiques comprenant de 1 à 12 atomes de carbone, R₅, R₆ représentant également l'atome d'hydrogène;
les composés organiques comportant au moins une double liaison éthylénique étant choisis parmi le butadiène, l'isoprène, l'hexadiène-1,5, le cyclooctadiène-1,5, le pentène-3-nitrile, le pentène-4-nitrile, le styrène, le méthylstyrène, le vinylnaphtalène, le cyclohexène, le méthylcyclohexène ainsi que les mélanges de plusieurs de ces composés.

2. Procédé selon la revendication 1, **caractérisé en ce que** le ligand organique est choisi dans le groupe comprenant les composes de formule suivante :

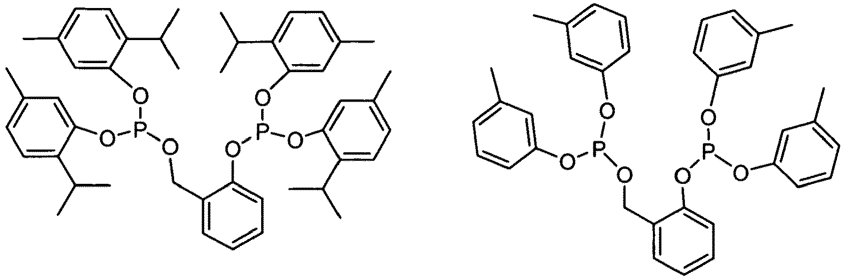

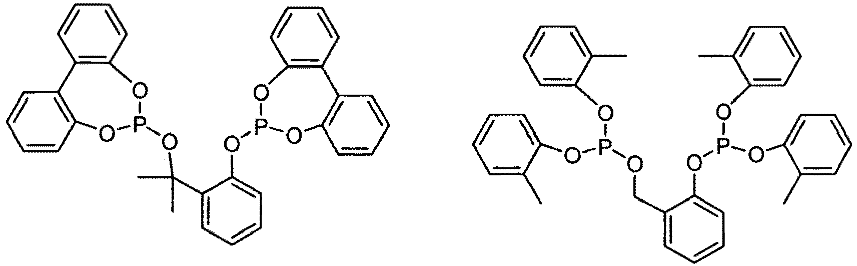

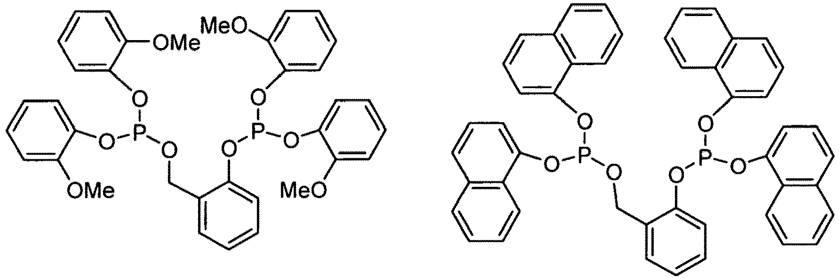

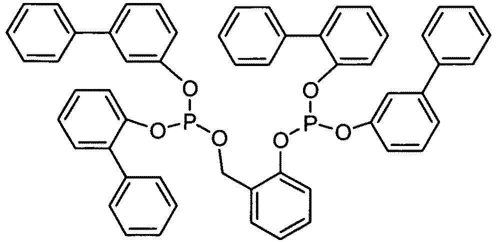

3. Procédé selon l'une des revendications précédentes, **caractérisé en ce que** la réaction est effectuée en milieu monophasique.

4. Procédé selon l'une des revendications précédentes **caractérisé en ce que** le catalyseur correspond à) a formule générale (V) :
M [Lf]ᵥ (V)
Dans laquelle:
M est le nickel
L_{f} représente le ligand organique de formule (I)
v représente un nombre compris entre 1 et 4 (bornes incluses).

5. Procédé selon l'une des revendications précédentes, **caractérisé en ce que** le milieu réactionnel comprend un solvant du catalyseur miscible à la phase comprenant le composé à hydrocyaner à la température d'hydrocyanation.

6. Procédé selon l'une des revendications précédentes, **caractérisé en ce que** les composés des métaux de transition sont ceux du nickel et sont choisis dans le groupe comprenant:
les composés dans lesquels le nickel est au degré d'oxydation zéro comme le tétracyanonickelate de potassium K₄[(Ni(CN)₄], le bis(acrylonitrile) nickel zéro, le bis (cyclooctadiène-1,5) nickel zéro et les dérivés contenant des ligands comme le tétrakis(triphényl-phosphine) nickel zéro;
les composés du nickel comme les carboxylates, carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

7. Procédé selon l'une des revendications précédentes, **caractérisé en ce que** la quantité de composé du nickel utilisée est choisie de telle sorte qu'il y ait par mole de composé organique à hydrocyaner entre 10⁻⁴ et 1 mole de nickel mis en oeuvre et **en ce que** la quantité de ligand organique de formule (I) utilisée est choisie de telle sorte que le nombre de moles de ce composé rapporté à 1 mole de nickel soit de 0,5 à 50.

8. Procédé selon l'une des revendications précédentes, **caractérisé en ce que** la réaction d'hydrocyanation est réalisée à une température de 10 °C à 200 °C.

9. Procédé selon l'une des revendications précédentes d'hydrocyanation en dinitriles de composés nitriles à insaturation éthylénique choisis parmi le pentène-3-nitrile, le pentène-4-nitrile, et leurs mélanges, par réaction avec le cyanure d'hydrogène, **caractérisé en ce que** l'on opère en présence d'un système catalytique comprenant au moins un composé du nickel, au moins un compose organique de formule (I) et un cocatalyseur consistant en au moins un acide de Lewis.

10. Procédé selon la revendication 9, **caractérisé en ce que** les pentènenitriles linéaires proviennent de la réaction antérieure d'hydrocyanation du butadiène et/ou de l'isomérisation du méthyl-2-butène-3-nitrile en pentènenitriles et contiennent des quantités d'autres composés choisis dans le groupe comprenant le méthyl-2-butène-3-nitrile, le méthyl-2-butène-2-nitrile, le pentène-2-nitrile, le valéronitrile, l'adiponitrile, le méthyl-2-glutaronitrile, l'éthyl-2-succinonitrile ou le butadiène.

11. Procédé selon l'une des revendications 9 ou 10, **caractérisé en ce que** l'acide de Lewis mis en oeuvre comme cocatalyseur est choisi parmi les composés des éléments des groupes Ib, IIb, IIIa, IIIb, IVa, IVb, Va, Vb, VIb, VIIb et VIII de la Classification périodique des éléments.

12. Procédé selon l'une des revendications 9 à 11, **caractérisé en ce que** l'acide de Lewis est choisi parmi les sels choisi dans le groupe des halogénures, sulfates, sulfonates, halogenoalkylsulfonates, perhalogénoalkylsulfonates, halogénoalkylacétates, perhalogénoalkylacétates, carboxylates et phosphates.

13. Procédé selon l'une des revendications 9 à 12, **caractérisé en ce que** l'acide de Lewis est choisi parmi le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, le trifluoroacétate d'indium, le trifluoroacétate de zinc, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium et leurs mélanges.

14. Procédé selon l'une des revendications 9 à 13, **caractérisé en ce que** l'acide de Lewis mis en oeuvre représente de 0,01 à 50 moles par mole de composé du nickel.

15. Procédé selon l'une des revendications 1 à 14, **caractérisé en ce que** l'on réalise en absence de cyanure d'hydrogène l'isomérisation en pentènenitriles, du méthyl-2-butène-3-nitrile présent dans le mélange réactionnel provenant de l'hydrocyanation du butadiène, en opérant en présence d'un catalyseur comportant au moins un ligand organique de formule générale (I) et au moins un composé du nickel.

16. Procédé selon la revendication 15, **caractérisé en ce que** le méthyl-2-butène-3-nitrile soumis à l'isomérisation est mis en oeuvre seul ou en mélange avec du méthyl-2-butène-2-nitrile, du pentène-4-nitrile, du pentène-3-nitrile, du pentène-2-nitrile, du butadiène, de l'adiponitrile, du méthyl-2-glutaroronitrile, de l'éthyl-2-succinonitrile ou du valéronitrile.

17. Procédé selon l'une des revendications 15 ou 16, **caractérisé en ce que** la réaction d'isomérisation est réalisée à une température de 10 °C à 200 °C.

18. Procédé selon l'une des revendications 15 à 17, **caractérisé en ce que** l'isomérisation en pentènenitriles du méthyl-2-butène-3-nitrile est réalisée en présence d'au moins un composé du nickel, d'au moins un ligand organique phosphoré de formule (I) et un cocatalyseur consistant en au moins un acide de Lewis.

## Patentansprüche

1. Verfahren zur Hydrocyanierung einer Kohlenwasserstoffverbindung, die mindestens eine ethylenische Ungesättigtheit umfasst, durch Umsetzung in flüssigem Medium mit Cyanwasserstoff in Gegenwart eines Katalysators, der ein Metallelement, bei dem es sich um Nickel handelt, und einen organischen Liganden umfasst, **dadurch gekennzeichnet, dass** der organische Ligand der folgenden allgemeinen Formel I entspricht: 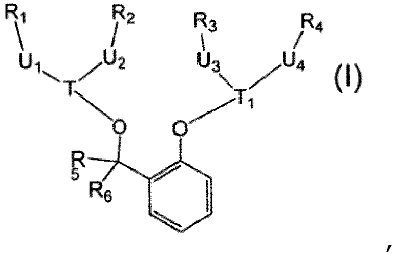 worin:
T, T₁, die gleich oder verschieden sind, für ein Phosphor-, Arsen- oder Antimonatom stehen
U₁, U₂, U₃, U₄, die gleich oder verschieden sind, für ein Sauerstoffatom oder einen Rest NR stehen, wobei R einen Alkyl-, Aryl-, Sulfonyl- oder Carbonylrest darstellt,
R₁, R₂, R₃, R₄, die gleich oder verschieden sind, für einen substituierten oder unsubstituierten, aromatischen, aliphatischen oder cycloaliphatischen Rest stehen, der einen oder mehrere Ringe in kondensierter oder nicht kondensierter Form umfasst und ein oder mehrere Heteroatome enthalten kann, wobei die Reste R₁ und R₂ einerseits sowie R₃ und R₄ andererseits miteinander durch eine kovalente Bindung, eine Kohlenwasserstoffkette oder ein Heteroatom verbunden sein können und, wenn einer der Reste U₁, U₂, U₃, U₄ ein Atom N enthält, der zugehörige Rest R₁, R₂, R₃, R₄ einen Ring bilden kann, der das Glied N des Restes einschließt, und
R₅, R₆, die gleich oder verschieden sind, für aliphatische, cycloaliphatische oder aromatische Reste stehen, die 1 bis 12 Kohlenstoffatome umfassen, wobei R₅, R₆ auch für ein Wasserstoffatom stehen;
wobei die organischen Verbindungen mit mindestens einer ethylenischen Doppelbindung aus Butadien, Isopren, 1,5-Hexadien, 1,5-Cyclooctadien, Pent-3-ennitril, Pent-4-ennitril, Styrol, Methylstyrol, Vinylnaphtalin, Cyclohexen, Methylcyclohexen sowie Gemischen von mehreren dieser Verbindungen ausgewählt werden.

2. Verfahren nach Anspruch 1, **dadurch gekennzeichnet, dass** der organische Ligand aus der Gruppe ausgewählt wird, die die Verbindungen der folgenden Formel umfasst:

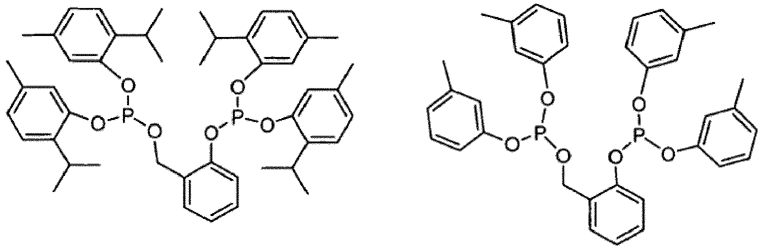

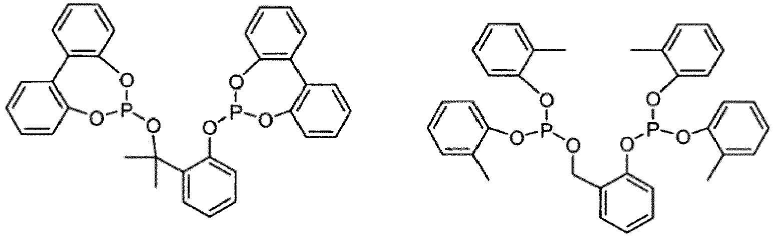

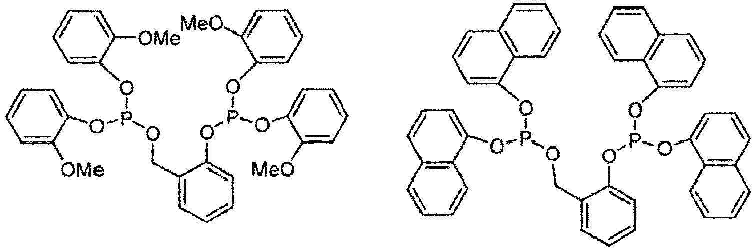

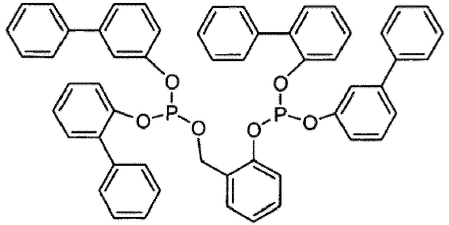

3. Verfahren nach einem der vorhergehenden Ansprüche, **dadurch gekennzeichnet, dass** die Reaktion in einem Ein-Phasen-Medium durchgeführt wird.

4. Verfahren nach einem der vorhergehenden Ansprüche, **dadurch gekennzeichnet, dass** der Katalysator der allgemeinen Formel (V) entspricht:
M [Lf]ᵥ (V),
worin:
M Nickel ist,
L_{f} für den organischen Liganden der Formel (I) steht
v eine Zahl zwischen 1 und 4 (einschließlich der Grenzen) darstellt.

5. Verfahren nach einem der vorhergehenden Ansprüche, **dadurch gekennzeichnet, dass** das Reaktionsmedium ein Lösungsmittel für den Katalysator umfasst, das mit der Phase, die die zu hydrocyanierende Verbindung umfasst, bei der Hydrocyanierungstemperatur mischbar ist.

6. Verfahren nach einem der vorhergehenden Ansprüche, **dadurch gekennzeichnet, dass** die Übergangsmetallverbindungen Verbindungen von Nickel und aus der Gruppe ausgewählt sind, die die Folgenden umfasst:
Verbindungen, in denen Nickel in der Oxidationsstufe null vorliegt, wie Kaliumtetracyanonickelat K₄[Ni(CN)₄], Bis(acrylnitril)nickel(0), Bis(1,5-cyclooctadien)nickel(0) und Derivaten, die Liganden wie Tetrakis(triphenylphosphin)nickel(0) enthalten;
Verbindungen von Nickel, wie Carboxylate, Carbonat, Bicarbonat, Borat, Bromid, Chlorid, Citrat, Thiocyanat, Cyanid, Formiat, Hydroxid, Hydrophosphit, Phosphit, Phosphat und Derivate, Iodid, Nitrat, Sulfat, Sulfit, Aryl- und Alkylsulfonate.

7. Verfahren nach einem der vorhergehenden Ansprüche, **dadurch gekennzeichnet, dass** die verwendete Menge an Nickelverbindung derart ausgewählt wird, dass pro Mol an zu hydrocyanierender organischer Verbindung zwischen 10⁻⁴ und 1 Mol eingesetzter Nickel vorliegt, und dadurch, dass die verwendete Menge an organischem Liganden der Formel (I) derart ausgewählt wird, dass die Anzahl der Mole dieser Verbindung, bezogen auf 1 Mol Nickel, 0,5 bis 50 beträgt.

8. Verfahren nach einem der vorhergehenden Ansprüche, **dadurch gekennzeichnet, dass** die Hydrocyanierungsreaktion bei einer Temperatur von 10°C bis 200°C durchgeführt wird.

9. Verfahren nach einem der vorhergehenden Ansprüche zur Hydrocyanierung von ethylenisch ungesättigten Nitrilverbindungen, ausgewählt aus Pent-3-ennitril, Pent-4-ennitril und ihren Gemischen, in Dinitrile durch Umsetzung mit Cyanwasserstoff, **dadurch gekennzeichnet, dass** man in Gegenwart eines Katalysatorsystems arbeitet, das mindestens eine Nickelverbindung, mindestens eine organische Verbindung der Formel (I) und einem aus mindestens einer Lewis-Säure bestehenden Co-Katalysator umfasst.

10. Verfahren nach Anspruch 9, **dadurch gekennzeichnet, dass** die geraden Pentennitrile aus einer vorherigen Hydrocyanierungsreaktion von Butadien und/oder der Isomerisierung von 2-Methylbut-3-ennitril in Pentennitrile stammen und Mengen anderer Verbindungen, ausgewählt aus der 2-Methylbut-3-ennitril, 2-Methylbut-2-ennitril, Pent-2-ennitril, Valeronitril, Adiponitril, 2-Methylglutaronitril, 2-Ethylsuccinonitril oder Butadien umfassenden Gruppe, enthalten.

11. Verfahren nach einem der Ansprüche 9 oder 10, **dadurch gekennzeichnet, dass** die als Co-Katalysator eingesetzte Lewis-Säure aus Verbindungen von Elementen der Gruppen Ib, IIb, IIIa, IIIb, IVa, IVb, Va, Vb, VIb, VIIB und VIII des Periodensystems der Elemente ausgewählt ist.

12. Verfahren nach einem der Ansprüche 9 bis 11, **dadurch gekennzeichnet, dass** die Lewis-Säure aus Salzen ausgewählt ist, die aus der Gruppe mit Halogeniden, Sulfaten, Sulfonaten, Halogenalkylsulfonaten, Perhalogenalkylsulfonaten, Halogenalkylacetaten, Perhalogenalkylacetaten, Carboxylaten und Phosphaten ausgewählt wird.

13. Verfahren nach einem der Ansprüche 9 bis 12, **dadurch gekennzeichnet, dass** die Lewis-Säure aus Zinkchlorid, Zinkbromid, Zinkiodid, Manganchlorid, Manganbromid, Cadmiumchlorid, Cadmiumbromid, Zinn(II)chlorid, Zinn(II)bromid, Zinn(II)sulfat, Zinn(II)tartrat, Indiumtrifluormethylsulfonat, Indiumtrifluoracetat, Zinktrifluoracetat, Chloriden oder Bromiden von Seltenerdeelementen, wie Lanthan, Cer, Praseodym, Neodym, Samarium, Europium, Gadolinium, Terbium, Dysprosium, Hafnium, Erbium, Thallium, Ytterbium und Lutetium, Kobaltchlorid, Eisenchlorid, Yttriumchlorid und ihren Gemischen ausgewählt wird.

14. Verfahren nach einem der Ansprüche 9 bis 13, **dadurch gekennzeichnet, dass** die eingesetzte Lewis-Säure 0,01 bis 50 Mole pro Mol an Nickelverbindung ausmacht.

15. Verfahren nach einem der Ansprüche 1 bis 14, **dadurch gekennzeichnet, dass** man in Abwesenheit von Cyanwasserstoff die Isomerisierung des im Reaktionsgemisch vorhandenen, aus der Hydrocyanierung von Butadien stammenden 2-Methylbut-3-ennitrils in Pentennitrile durchführt, indem man in Gegenwart eines Katalysators arbeitet, der mindestens einen organischen Liganden der allgemeinen Formel (I) und mindestens eine Nickelverbindung enthält.

16. Verfahren nach Anspruch 15, **dadurch gekennzeichnet, dass** das 2-Methylbut-3-ennitril, das der Isomerisierung unterzogen wird, allein oder im Gemisch mit 2-Methylbut-2-ennitril, Pent-4-ennitril, Pent-3-ennitril, Pent-2-ennitril, Butadien, Adiponitril, 2-Methylglutaronitril, 2-Ethylsuccinonitril oder Valeronitril eingesetzt wird.

17. Verfahren nach einem der Ansprüche 15 oder 16, **dadurch gekennzeichnet, dass** die Isomerisierungsreaktion bei einer Temperatur von 10°C bis 200°C durchgeführt wird.

18. Verfahren nach einem der Ansprüche 15 bis 17, **dadurch gekennzeichnet, dass** die Isomerisierung von 2-Methylbut-3-ennitril in Pentennitrile in Gegenwart von mindestens einer Nickelverbindung, mindestens einem phosphorhaltigen organischen Liganden der Formel (I) und einem Co-Katalysator, der aus mindestens einer Lewis-Säure besteht, durchgeführt wird.

## Claims

1. Process for the hydrocyanation of a hydrocarbon compound comprising at least one ethylenic unsaturation by reaction, in a liquid medium, with hydrogen cyanide in the presence of a catalyst comprising a metallic element which is nickel and an organic ligand, **characterized in that** the organic ligand corresponds to the general formula I below: 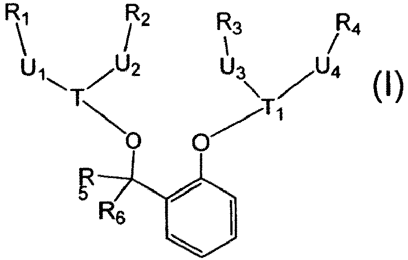 in which:
T and T₁, which are identical or different, represent a phosphorus, arsenic or antimony atom,
U₁, U₂, U₃ and U₄, which are identical or different, represent an oxygen atom or a radical NR, R representing an alkyl, aryl, sulphonyl or carbonyl radical,
R₁, R₂, R₃ and R₄, which are identical or different, represent a substituted or unsubstituted aromatic, aliphatic or cycloaliphatic radical comprising one or more rings, which are optionally in fused form and which may comprise one or more heteroatoms, the radicals R₁ and R₂ on the one hand and R₃ and R₄ on the other hand may be connected to one another by a covalent bond, a hydrocarbon chain or a heteroatom, and, when one of the radicals U₁, U₂, U₃ and U₄ comprises an N atom, the associated radical R₁, R₂, R₃ or R₄ may form a ring including the N element of said radical, and
R₅ and R₆, which are identical or different, represent aliphatic, cycloaliphatic or aromatic hydrocarbon radicals comprising from 1 to 12 carbon atoms, R₅ and R₆ also representing the hydrogen atom;
the organic compounds comprising at least one ethylenic double bond being selected from butadiene, isoprene, 1,5-hexadiene, 1,5-cyclooctadiene, 3-pentenenitrile, 4-pentenenitrile, styrene, methylstyrene, vinylnaphthalene, cyclohexene, methylcyclohexene and also mixtures of several of these compounds.

2. Process according to Claim 1, **characterized in that** the organic ligand is selected from the group comprising the compounds of formula below:

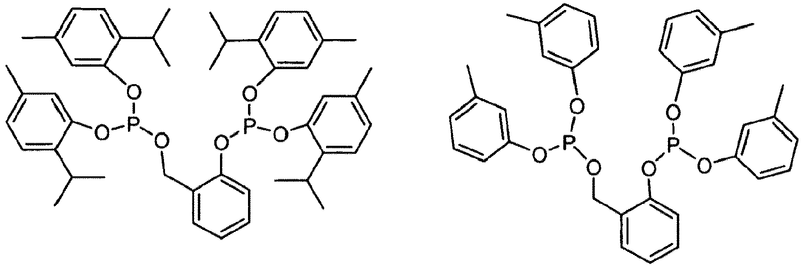

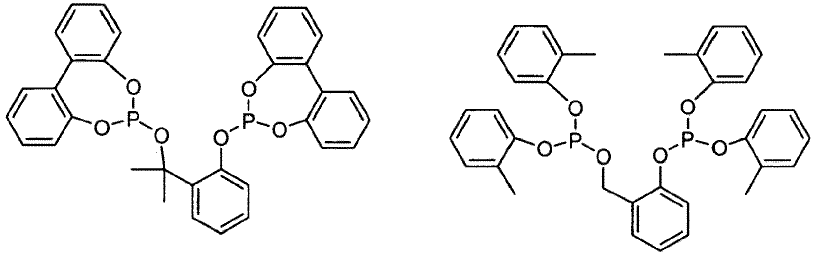

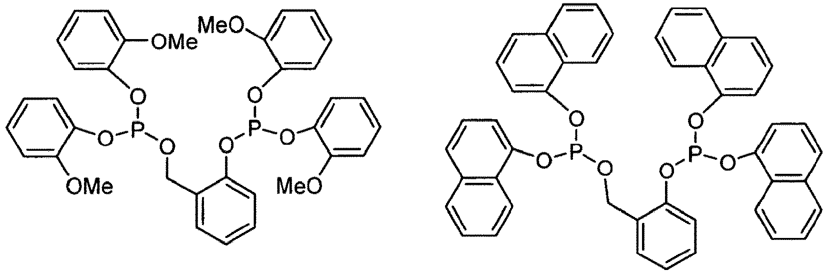

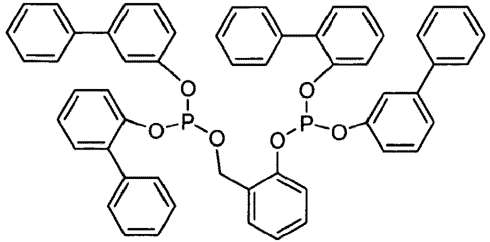

3. Process according to either of the preceding claims, **characterized in that** the reaction is carried out in a single-phase medium.

4. Process according to one of the preceding claims, **characterized in that** the catalyst corresponds to the general formula (V):
M[Lf]ᵥ (V)
in which:
M is nickel,
L_{f} represents the organic ligand of formula (I) and
v represents a number between 1 and 4 (inclusive).

5. Process according to one of the preceding claims, **characterized in that** the reaction mixture comprises a solvent for the catalyst which is miscible with the phase comprising the compound to be hydrocyanated at the hydrocyanation temperature.

6. Process according to one of the preceding claims, **characterized in that** the transition metal compounds are nickel compounds and are selected from the group comprising:
compounds in which nickel is in the zero oxidation state, such as potassium tetracyanonickelate K₄[Ni(CN)₄], bis(acrylonitrile)nickel (0), bis(1,5-cyclooctadiene)nickel (0) and derivatives containing ligands such as tetrakis(triphenylphosphine)nickel (0);
nickel compounds such as carboxylates, carbonate, bicarbonate, borate, bromide, chloride, citrate, thiocyanate, cyanide, formate, hydroxide, hydrophosphite, phosphite, phosphate and derivatives, iodide, nitrate, sulphate, sulphite, aryl- and alkylsulphonates.

7. Process according to one of the preceding claims, **characterized in that** the amount of nickel compound used is selected such that there is, per mole of organic compound to be hydrocyanated, between 10⁻⁴ and 1 mol of nickel used and **in that** the amount of organic ligand of formula (I) used is selected such that the number of moles of this compound relative to 1 mol of nickel is from 0.5 to 50.

8. Process according to one of the preceding claims, **characterized in that** the hydrocyanation reaction is carried out at a temperature from 10°C to 200°C.

9. Process according to one of the preceding claims for hydrocyanation of ethylenically unsaturated nitrile compounds selected from 3-pentenenitrile, 4-pentenenitrile and mixtures thereof to give dinitriles, by reaction with hydrogen cyanide, **characterized in that** it is carried out in the presence of a catalytic system comprising at least one nickel compound, at least one organic compound of formula (I) and a cocatalyst consisting of at least one Lewis acid.

10. Process according to Claim 9, **characterized in that** the linear pentenenitriles originate from the prior hydrocyanation reaction of butadiene and/or from the isomerization of 2-methyl-3-butenenitrile to give pentenenitriles and contain amounts of other compounds selected from the group comprising 2-methyl-3-butenenitrile, 2-methyl-2-butenenitrile, 2-pentenenitrile, valeronitrile, adiponitrile, 2-methylglutaronitrile, 2-ethylsuccinonitrile or butadiene.

11. Process according to either of Claims 9 and 10, **characterized in that** the Lewis acid used as cocatalyst is selected from compounds of the elements of groups Ib, IIb, IIIa, IIIb, IVa, IVb, Va, Vb, VIb, VIIb and VIII of the Periodic Table of the Elements.

12. Process according to one of Claims 9 to 11, **characterized in that** the Lewis acid is selected from salts selected from the group of halides, sulphates, sulphonates, haloalkylsulphonates, perhaloalkylsulphonates, haloalkylacetates, perhaloalkylacetates, carboxylates and phosphates.

13. Process according to one of Claims 9 to 12, **characterized in that** the Lewis acid is selected from zinc chloride, zinc bromide, zinc iodide, manganese chloride, manganese bromide, cadmium chloride, cadmium bromide, stannous chloride, stannous bromide, stannous sulphate, stannous tartrate, indium trifluoromethylsulphonate, indium trifluoroacetate, zinc trifluoroacetate, the chlorides or bromides of rare earth elements such as lanthanum, cerium, praseodymium, neodymium, samarium, europium, gadolinium, terbium, dysprosium, hafnium, erbium, thallium, ytterbium and lutetium, and cobalt chloride, ferrous chloride, yttrium chloride and mixtures thereof.

14. Process according to one of Claims 9 to 13, **characterized in that** the Lewis acid used represents from 0.01 to 50 mol per mole of nickel compound.

15. Process according to one of Claims 1 to 14, **characterized in that** 2-methyl-3-butenenitrile, present in the reaction mixture originating from the hydrocyanation of butadiene, is isomerized in the absence of hydrogen cyanide to give pentenenitriles, in the presence of a catalyst comprising at least one organic ligand of general formula (I) and at least one nickel compound.

16. Process according to Claim 15, **characterized in that** the 2-methyl-3-butenenitrile subjected to isomerization is used alone or in a mixture with 2-methyl-2-butenenitrile, 4-pentenenitrile, 3-pentenenitrile, 2-pentenenitrile, butadiene, adiponitrile, 2-methylglutaronitrile, 2-ethylsuccinonitrile or valeronitrile.

17. Process according to either of Claims 15 and 16, **characterized in that** the isomerization reaction is carried out at a temperature from 10°C to 200°C.

18. Process according to Claims 15 to 17, **characterized in that** the isomerization of 2-methyl-3-butenenitrile to give pentenenitriles is carried out in the presence of at least one nickel compound, at least one organic phosphorus-containing ligand of formula (I) and a cocatalyst consisting of at least one Lewis acid.
